Europäisches Patentamt

European Patent Office

Office européen des brevets

(11) Publication number : **0 570 089 A1**

(12) **EUROPEAN PATENT APPLICATION**

(21) Application number : 93250133.1

(22) Date of filing : 11.05.93

(51) Int. Cl.⁵ : **A01N 63/04,** A01N 35/06,
C12N 1/14, // (C12N1/14,
C12R1:645)

(30) Priority : **13.05.92 US 882335**

(43) Date of publication of application :
**18.11.93 Bulletin 93/46**

(84) Designated Contracting States :
**DE ES FR GB IT NL**

(71) Applicant : **W.R. Grace & Co.-Conn.**
**Grace Plaza, 1114 Avenue of the Americas**
**New York, New York 10036-7794 (US)**

(72) Inventor : **Eyal, Jacob**
**23, Emerald Ridge Court**
**Baltimore, Md. 21209 (US)**
Inventor : **Fischbein, Karen Louise**
**8625 Houlton Harbour**
**Pasadena, MD 21122 (US)**
Inventor : **Walter, James Frederic**
**1008 Ashland Drive**
**Ashton, MD 20861 (US)**

(74) Representative : **UEXKÜLL & STOLBERG**
**Patentanwälte**
**Beselerstrasse 4**
**D-22607 Hamburg (DE)**

(54) **Novel toxin producing fungal pathogen and uses.**

(57)   A novel toxin-producing isolate of the fungus *Beauveria bassiana* which produces metabolities, mainly oosporin and analogs thereof, is disclosed. Biopesticides prepared from a sporulated biomass produced by submerged fermentation of the fungus in combination with the toxic metabolites produced by the fungus provide high virulency for control of pests. The novel fungus is *Beauveria bassiana* ATCC 74037. Also disclosed is a method of using the fungus to produce the toxin oosporin, and analogs thereof, in high yields using a submerged fermentation process.

EP 0 570 089 A1

Jouve, 18, rue Saint-Denis, 75001 PARIS

FIELD OF THE INVENTION

This invention relates to a novel, highly virulent, entomogenic fungus isolate of *Beauveria bassiana* and metabolites therefrom. The novel isolate is characterized by production of highly pathogenic spores and substantial amounts of toxic red pigment during growth. The combination of pathogenic spores and toxic red pigment acts synergistically to produce a highly effective biocontrol agent against pests.

This invention also relates to a process of formulating, delivering and using the novel toxin producing isolate of *Beauveria bassiana* as a biopesticidal agent for the effective control of soil and plant insects.

## BACKGROUND OF THE INVENTION

Chemical pesticides have been used extensively to control pests for many years. An awareness of recent problems in the use of pesticides and concern about their adverse effects on man and his environment have resulted in more commercial attention being given to biological control alternatives.

Certain entomogenic fungi have been recognized by researchers to be pathogenic to different pests. Entomogenic fungi have been explored widely for use as biocontrol agents. Update reviews on the use of different pathogenic fungi can be found in Carlo M. Ignoffo and B. Handaua "*Handbook of Natural Pesticides*", Vol. V, part A., C. W. McCoy et al., *Fungi in Biological Control Systems"*, Manchester University Press, 1988. Entomogenic fungi such as *Metarhizium, Hirsutella, Vermicilum, Paecilomyces,* and other species have been studied for use as pest control agents.

Attention has been given to the entomogenic fungi *Beauveria bassiana (Bols) Vuill,* which is found naturally on many disease insects, as a pest biocontrol agent. In particular, a species of *Beauveria bassiana* has been used in the field as a mycoinsecticide against two agriculturally important pests, the Colorado potato beetle, Hajek et al., Can. Entomol., Vol. 119, 959-974 (1987); T.E. Anderson et al., J. Econ. Entomol., Vol. 82, 83-89 (1989); and Feng et al., Can. Entomol., Vol. 120, 133-144 (1988), and the grasshopper, S. Marcandier et al., Can. Entomol., Vol. 119, 901-907 (1987); and K.C. Moore et al., Can. Entomol., Vol. 120, 989-991 (1988). U.S. Patent 4,925,663 (Stimac) discloses the use of an isolate of *Beauveria bassiana* for the biological control of imported fire ants.

The genus *Beauveria* is known to produce different color pigments and secondary metabolites (J.E. Zajic, J. Insect. Pathol., Vol. 5(1), 1963) (Walt et al., Lan J. Chem., Vol. 55, 1977). Several studies have examined the different pigments of *Beauveria.* Yellow pigments produced by *Beauveria bassiana* were identified as tenellin and bassianin. (Chi-Kit Wat et al., Can. J. Chem., Vol. 55, 1977). One of the pigment metobilites which has been identified in *Beauveria bassiana* is oosporin. (F. Vining et al., Can. J. of Botany, Vol. 46, 1968). However, red pigment production is not a constant characteristic of the genus *Beauveria* and most of the *Beauveria* culture do not produce a red pigment or loses the ability to produce red pigment after subculture.

Oosporin is a toxic red pigment that was originally isolated from *Oospora colorants* by Kogl and Van Wessem in 1944. Cole characterized oosporin from a fungi named *Chaetomium trilaterale* for chemical and physical properties (J. Agr. Food Chem. (1974) 22:3). Oosporin has been extensively studied due to its toxic characteristics on plant and poultry, (Brown et al., Avian Diseases (1987) 31:868-877, Pegram Avian Diseases (1981) 26:1) This red pigment has also been investigated for its antibiotic properties for treatment of animal and human bacterial diseases (Wainwright et al., Trans. Br. Myco. Soc. (1986) 86:1). *Beauveria bassiana* producing the toxin, oosporin or analogs thereof, have not heretofore been used as a biological control agent against pests and insects.

## SUMMARY OF THE INVENTION

This invention provides a novel toxin producing isolate of the fungus *Beauveria bassiana* which has high virulency for the control of pests. The novel *Beauveria bassiana* isolate of this invention has the capability to produce metabolites, mainly oosporin analogs, in high yield and retains this capability after subculture. Conidial spores of the novel fungus can be used in combination with oosporin produced by the fungus to provide effective control of soil and plant insects, as well as nematodes.

The novel *Beauveria bassiana* isolate of the invention can be produced by submerged culture. The formulated biopesticide is formed into a prilled product which is easily handled and applied. By using a formulated prill, biological activity of the product is maintained until the time of application. Without prill formulation, the fungus could not survive under storage conditions. Upon application to soil, seed, root, or plant and upon rewetting, the dried prill is "reactivated", that is reconverted into a biologically active form. Mycelium budding from the prill consumes the nutrients provided by the prill formulation and conidialtion begins, resulting in the production of conidial spores and oosporin. Conidial spores are the biologically active form of the fungus which is pathogenic to pests.

The prilled biopesticide agent of the invention can be easily produced, handled, stored, shipped, and applied for controlling plant and soil pests. The prilled product can be stored at about room temperature, preferably between 4°C to 25°C for extended periods, i.e., for more than a year. A storage temperature of about 4°C to about 10°C is most preferred in order to avoid losing condiation activity and spore viability.

A primary object of this invention is to provide a novel toxin producing isolate of *Beauveria bassiana.*

It is also an object of this invention to provide a novel isolate of *Beauveria bassiana* which produces oosporin, and analogs thereof, in high yield even after subculture.

It is another object of this invention to provide a prilled biopesticidal product containing the toxin producing isolate of *Beauveria bassiana,* which prilled product promotes viability of the fungus during storage and can be readily mass produced as required for horticultural and agricultural applications.

It is also an object of this invention to provide an economical, commercial-feasible process for producing a biopesticide product containing the toxin producing isolate of *Beauveria bassiana.* The process produces a biocontrol material that is easy to handle and to apply in either horticulture or agriculture settings.

It is a further object of this invention to provide a method for using the toxin producing isolate of *Beauveria bassiana* as a safe biopesticidal agent which has a high level of infectivity for various soil and plant pests.

Still another object of this invention is to provide a process for converting an actively growing culture of the novel toxin producing fungus into a formulated biopesticide.

Another object of this invention is to provide a novel biopesticidal formulation that is useful in the prevention or control of pest infestation including, but not limited to, plant and soil-borne insects and nematodes.

An additional object of the invention is to provide an alternative to chemical pesticides.

It is yet another object of the present invention to provide a method of producing the pigment oosporin, and analogs thereof, in large amounts using a fermentation process. This process permits the large scale production of oosporin without the use of chemical synthesis.

## DETAILED DESCRIPTION OF THE INVENTION

For purposes of the invention, the term "biopesticide" shall mean an entomogenic fungi which, after formulating in a prill, powder, or suspension form, is used in the control or prevention of plant and soil pests. The control or prevention is accomplished by adversely effecting the existence or growth of a target pest, particularly insects. Such control can comprise a complete killing action, eradication, arrest of growth, reduction in number, or any combination of these actions. The term "entomogenic" as used in the specification and claims of this invention shall mean pathogenic to insects specifically, but can also be construed broadly to mean pathogenic to pests. The term "control" or "biocontrol" as employed in the specification and claims of this invention shall mean protecting plants or soil from pest damage by use of the biopesticides of this invention. The term "pesticidally effective amount" or "effective amount" is used herein to indicate that amount of biopesticide sufficient to exert pest control upon a targeted pest.

### The Culture

A biologically-pure culture of a novel toxin-producing isolate of *Beauveria bassiana* of the present invention has been deposited in the American Type Culture collection (ATCC), 12301 Parklawn Drive, Rockville, MD 20852. The culture has been given the accession number ATCC 74037.

The culture was deposited under conditions that assure that access to the culture will be available during the pendency of the patent application to be determined by the Commissioner of Patents and Trademarks to be entitled thereto under 37 C.F.R. 1.144 and 35 U.S.C. 112. The deposit is available as required by foreign patent laws in countries wherein counterparts of the subject application, or its progeny, are filed. Further, the culture deposit will be stored and made available to the public in accord with the provisions of the Budapest Treaty for the deposit of microorganism.

The *Beauveria bassiana* ATCC 74037 was isolated and purified from a sweet potato white fly using conventional methodology. The taxonomy of the fungus include conidiogenous cells either in clusters or irregularly scattered on aerial hypal conidial globose, subglobose or sometimes ellipsoidal in pure culture, hyaline smooth walled 2-3 μm in diameter. This taxonomic description is the same as that for other members of the species, *Beauveria bassiana.*

The novel *Beauveria bassiana* isolate of the invention differs from other members of the *Beauveria* species metabolically and biochemically. *Beauveria bassiana* ATCC 74037 is highly virulent against a large number of relevant pests, and also produces a high amount of toxic red pigment (oosporin and oosporin analogs) during growth and infection of the insect. The isolate grows at an unusually low temperature (i.e., about 4 to about 10°C) and still produces spores and toxins.

## Fermentation

The biopesticidal product of the invention can be prepared by growing *Beauveria bassiana* ATCC 74037 on a suitable media using standard surface culture methods. Suitable media include, but are not limited to, potato dextrose agar (PDA), V-8™ juice agar, lima bean agar, yeast extract medium, oatmeal agar and mixed cereal agar. Preferably, the medium is yeast extract media. The culture is incubated at a temperature and for a period of time sufficient to produce blastospores and mycelium which will be used for inoculating fermentors. Preferably the inoculum culture is incubated at a temperature of about 25°C to about 28°C for 1 to 2 days. This culture is used to inoculate fermentors using standard methods. As will be obvious to one skilled in the fermentation art, other inoculation methods may also be used.

The fermentation should be conducted in a manner such that the biomass in the fermentor substantially comprises filamentous mycelium, i.e., at least about 80% mycelium. This can be achieved by supplying an excess of complex carbon and complex nitrogen sources. The complex carbon source can be naturally occurring mixtures such as molasses, and the complex nitrogen source can be corn steep liquor and/or cotton seed flour. Other known complex carbon and complex nitrogen sources can also be used. Supply of pure sugars such as sucrose, dextrose, glucose or other sugar sources will result in the formation of mainly blastospores and are therefore not preferred. The composition of the nutrient medium can be varied over a wide range. However, the preferred nutrient solution may contain about 4 to 8 wt% complex carbon source and about 0.5 to 5 wt% complex nitrogen source. The production of mycelium can also be intensified by the constant supply (fed batch addition) of the complex carbon source, i.e., molasses, during the fermentation process. The fermentation can be carried out in batch, continuous or in fed batch mode.

In order to increase the yield of filamentous mycelium in the fermentor, it is desirable to add stimulant nutrients to the cultivation media. Such stimulant nutrients include, for example, liquid fat, oils, surfactants, and polyacids such as linoleic acid, silicon oils, water emulsion, etc.

A fed batch fermentation process can be advantageous to use, especially when the pH of the fermentation is shifting toward acid conditions. By adding molasses or other suitable complex carbon sources, it is possible to maintain constant conditions in the fermentation without using large amounts of base for pH adjustment. Fed batch addition of molasses, for example, can be used as buffer control for the fermentation. Preferably, the addition of the complex carbon source during fermentation is at a rate sufficient to maintain the carbon source at a level of at least about 1 percent by weight of the nutrient solution. Preferably, molasses addition begins after about 48 hours or when the pH shifts toward acidic conditions, i.e., below about pH 5.0.

The fermentation should be carried out in such a manner that agitation and aeration will be maximized. Any convenient means of aeration and agitation can be employed. If agitation is low, aggregation of mycelium occurs. Therefore a minimum agitation rate of about 400 rpm to 600 rpm is preferred with aeration in the order of about 0.8 to 1 vvm. The fermentation process should be carried out in the temperature range of about 20 to 30°C, preferably about 28 to 30°C, most preferably about 28°C for about 5 to 7 days.

The adjustment of the pH value is necessary in order to achieve formation of mycelium. Most favorable is pH 4.0 to 6.0, preferably about 5.0. Adjustment and control of the pH value can be achieved by the addition of an organic or inorganic base, preferably sodium hydroxide, ammonium hydroxide or any solution of triethyl amines.

In order to prevent developing undesirable amounts of foam during fermentation, standard chemical defoaming agents can be added. Standard chemical defoaming agents include silicon oil, polypropylene or glycol compounds, or other synthetic antifoams. The end of the cultivation can be easily determined by the standard method of biomass determination (e.g., dry mass determination). A 96-hour cultivation typically will be sufficient to yield 30-50 g/liter of dry mycelium. This yield is sufficient for use in the formulation stage of this invention.

The separation of the filamentous mycelium biomass from the fermentation media can be accomplished by standard procedures such as filtration, centrifugation or other convenient means of separation. To avoid contaminating the mycelium with undesirable microorganisms, harvesting the mycelium under sterile conditions is recommended.

## Formulation

*Beauveria bassiana* isolate ATCC 74037 may be formulated with a suitable agricultural carrier and optionally nutrients to provide a biopesticidal composition. The carrier should be an inert substance and may be in a liquid, powder, granulate or particulate form. For increased stability, the preferred formulation comprises a biomass of the fungus incorporated with a carrier and nutrients into a prill form. For purposes of this invention the term "biomass" is used to designate mycelium of *Beauveria bassiana.*

The carrier used in the biopesticidal formulation should be a carrier which is sufficient to promote or support growth of the fungal material. Suitable carriers include inert filling compounds such as clay, bentonite, talcum, perlite, peatmoss, diatomaceous earth, kaolin, vermiculite, bran, dry milk, and minerals. Preferably, the carrier is vermiculite. The carrier is preferably pretreated prior to use to reduce the level of contaminating microorganisms. This preferably is accomplished by heating (i.e., at 100°C or higher for at least up to about 1 hour) or alternatively, may be accomplished by irradiation. Microbial decontamination may be also accomplished chemically, provided that there is no residual chemical interference with fungal viability and growth. Returning to the preferred embodiment, the filamentous mycelium biomass is then added to the vermiculite or other carrier component by mixing.

Nutrients can be included in the mycelium formulation. Such nutrient sources may include carbon and nitrogen sources such as molasses, whey, milk powder, different autolyzed peptone, bran, wheat, malt extract or yeast extracts. It may be desirable to add stabilization and/or protective agents to the formulation, such as, for example, polyalcohols, glycerin or sugars. Antioxidants compounds such as ascorbic acid or propyl gallate may also be added, if desired.

The formulated mycelium mixture is then prilled by methods known to one skilled in the art. The prilling process can be conducted by adding a prilling agent such as, for example, sodium alginate, potassium alginate or the like, to the biomass/carrier mixture and by dropwise adding the mixture to a bath containing a coagulant such as, for example, calcium chloride or calcium gluconate. The concentration of the prilling agent in the propagule mixture can vary from about 0.2 to about 3% of the mixture depending on which formulation is used and degree of propagulation needed. The concentration of the coagulant in the bath can vary from about 1 to about 15% w/v as needed for suitable prill formation. Coagulation proceeds faster when the concentration of salts in the coagulation bath is increased. The prill formed by this method can be dried immediately by using any convenient drying method, such as, for example, air drying or oven drying. To obtain a flowable prill with uniform, physical characteristics (i.e., shape, mechanical strength, size, and density), it is preferable to use a fluid bed process.

In order to increase the conidialtion potential of the prill, it may be desirable to add nutrients to the prill to stimulate conidialtion of the fungal material. Such stimulating material can contain natural food ingredients such as molasses, peptone, glucose solution, etc. The addition of the nutrient can be before the drying process or during the drying process. Addition of nutrients before drying can be achieved, for example, by submerging the prill in a concentrated nutrient solution until diffusion is complete and then drying the treated prill. Addition of nutrients during the drying process can be achieved, for example, by coating the prill during their movement in the fluidized bed. The prill should be dried to a moisture or total volatiles content of about 2 to about 12% (w/w). However, a total volatiles content of about 6-8% is preferred. The moisture content can be easily determined according to standard methodology using moisture balances. To avoid contamination with undesirable microorganisms, the drying process is preferably done in sterile air, for example, by using air filters.

Prill produced according to the invention can be stored under dry conditions at temperatures between about 4°C to about 25°C. To preserve the viability of the prill beyond 12 months, it is preferable to store the prill in a vacuum or in an inert atmosphere such as argon, nitrogen or other inert gases.

For purposes of this invention the term "prill" is used to indicate a stable, granulated particle or bead which does not produce a powder or dust and which has an average particle size of from about 0.1 mm to about 5 mm in diameter, preferably about 1 mm to about 2 mm in diameter.

## Reactivation and Harvest

The formulated prill can optionally be activated, prior to use, to produce a concentrate of highly active, fresh conidial spores. Reactivation of the formulated prill produces of a large number of spores in a short time. Each prill can produce as much as $10^7$-$10^8$ conidial spores per prill and the conidial spores have high insecticidal effect on controlling pests. Reactivation is easily achieved by addition of moisture, preferably water, to the prill and allowing the growth of mycelia and production of conidial spores. For optimal growth conditions, incubation is run at about 20-30°C, preferably about 25°C for less than about seven days, preferably about 3 to about 4 days. Reactivation can take place in any suitable closed, sterile container, such as a petri dish or tray. The prill preferably should be placed as a monolayer in the container in order to achieve maximum surface area for the sporulation and germination process. The reactivated prill can be stored for prolonged periods of time, i.e., up to about 1 year at about 4°C to about 6°C, without losing conidialtion activity or spore viability.

The conidial spores can be easily harvested from the reactivated prill by washing with water or a non-toxic oil to provide aqueous or oil suspensions of conidial spores. Preferably, the aqueous suspension contains a surfactant. Any suitable surfactant can be used, such as, for example, a polyoxyethylene sorbitan monolaurate surfactant sold under tradenames Tween 80® or Tween 20® by Fisher Scientific of Fairlawn, New Jersey, in the range of 0.01 - 0.1%. Suitable oils include any oil which is non-toxic such as, for example, cottonseed oil, vegetable oil, peanut oil, soybean oil, palm oil, sesame oil, jojoba oil, coconut oil, mineral oil or any other edible, non-toxic oil. Preferably, the oil is cottonseed oil. Conidial spores harvested by water can be used immediately or stored until application preferably, under cool conditions of about 4°C. Conidial spores harvested by oil can be stored for up to a year, preferably about 6 months at room temperature (~25°C). In general, liquid suspensions having conidial spores in the amount of about $1 \times 10^9$ to $1 \times 10^{10}$ spores per ml of liquid may be obtained by cultivation and harvesting of the prill in accordance with the process of the invention. For purposes of the invention the term "non-toxic oil" is used to mean any oil which does not have any adverse or chronic toxicological properties to humans or the fungal material.

It is also within the scope of the invention to add nutrients to the aqueous suspension or non-aqueous oil suspension of conidial spores before application to the targeted area of treatment. Such nutrients can enhance the germination of the conidial spores on the pests. Alternatively, it can be useful to pregerminate the conidial spores prior to application. Such pregermination can be easily established by stirring the harvested conidial spores in water for about 4-8 hours in a container or vessel or any other suitable means.

In another embodiment of the invention, the reactivated prill having the conidial spores attached thereto may be stored or packaged in a water-soluble polymeric container prior to harvesting the spores. To harvest the conidial spores from a reactivated prill so packed, the water-soluble polymeric container having the activated prill contained therein is dissolved in water and the resulting emulsion is filtered to provide an aqueous suspension of conidial spores. Any polymeric material which is soluble in water, is inert to the fungal matter, and is capable of forming a container may be employed in the invention. Preferably, the polymeric material is a polyvinyl alcohol. The polymeric materials may be formed into a container using conventional methodology known in the container or packaging arts, such as, for example, by heat-sealing.

## Use

The application of the *Beauveria bassiana* biopesticide product prepared according to the invention is dependent on the nature of the pest to be controlled and the particular application (agriculture, horticulture, or forest). If the pest target is a plant pest, the biopesticide can be applied by spreading in a location near the plant or on the plant. If the target is a soil pest, the biopesticide can be applied to or mixed with the soil. Pests that can be controlled by the biopesticide of this invention include arthropods, nematodes, and particularly insects belonging to the arachnid family. The biopesticides are effective against pests which are normally sensitive and also those which are resistant to conventional pesticides. They are effective against all or individual pest development stages. The biopesticides can be used effectively against pests from the following orders: *Isopoda, Oniscus asellus, Armadillidium, Diplopoda, Chilopoda, Symphyla, Thysanura, Collembola, Orthopera, Dermaptera, Isoptera, Anoplura, Mallophage, Thysanoptera, Heteroptera, Homoptera, Lepidoptera, Colaoptera, Hymenoptera, Diptera, Siphonaptera, Arachnida,* and *Acarina.*

Also, the biopesticides can be used against plant parasitic nematodes including *Meloidogyne spp., Pratylenchus spp., Radopholus similes, Ditylanchus dipsaci, Heterodera spp., Xiphenema spp.,* and *Hoplolaemus spp. (loboder rostochiensis, meloidogyne spp.* (root-knot nematode), *Globodera rostochiensis* (potato cyst nematode), *Tylenchulus semipenetrans* (citrus nematode), *Radopholus similis* (burrowing nematode), *Rotylenchulus reniformis* (reniform nematode), *Pratylenchus spp.* (lesion nematode), *Helicotylenchus spp.* (spiral nematode), *Tylenchorhynchus spp.* (stunt nematode), *Trichodorus spp.* (stubby nematode), *Rotylenchus spp.,* and *Hoplolaimus sp.*

The principle target insect groups which are preferred for the biopesticides of this invention are: *Aphidae* (aphids), *Delphacidal* (planthoppers), *Cicadellidae* (leafhoppers), *Cercopidae* (spittlebugs), *Aleyodidae* (white fly), *Coccidea* (scales), *Thysaoptera* (thrips), *Coleoptera* (beetles), and *Lepidoptera* (caterpillars). Also targeted are mealy bugs, spider mites and other foliage insects.

The biopesticide dosage will vary greatly depending on the application. Factors to consider include the kind of biopesticidal formulation used, i.e., prill, spore suspension, etc., the kind of pest, the state of crop infested with the pest, the prevailing weather conditions, and the kind of the agriculture area (e.g., agriculture, horticulture, frosty or other conditions). In general, application of between about 20-60 kg bioinsecticide per hectare are recommended. The biopesticides can be applied by any convenient application method including, for example, broad cast spreading on the soil or plant mixture with the soil or spraying.

Oosporin Production

The novel *Beauveria bassiani* isolate ATCC 74037 possess a capacity for production of the toxic pigment, oosporin, in high yields during submerged fermentation as described hereinabove. A solid or liquid medium may be used for pigment production. An aerobic cultivation in a liquid medium is prefered to promote maximum production and recovery of oosporin. The novel *Beauveria* of the invention produces a higher amount of oosporin than prior known pigment producing fungi. As mentioned hereinabove, fermentation of *Beauveria bassiana* isolate ATCC 74037 is conducted in the presence of excess complex carbon and complex nitrogen nutrient sources. During the course of fermentation, an excess or high carbon source is maintained by direct replenishment while the nitrogen source is allowed to be exhausted. Upon exhaustion of the nitrogen source the *Beauveria* mycelium spontaneously begins to connect the culture medium to produce and accumulate oosporin, extracellularly in the fermentation medium. Upon extraction of the *Beauveria* mycelium, no pigment was determined to be produced intracellularly in the walls of the fungal mycelia. The release of oosporin in the culture medium is evidenced by the appearance of a red color in the medium, which color changes to a dark red color as more pigment is released. Usually red pigment began to appear in the fermentation medium after about 24 to 36 hours of fermentation. Recovery of oosporin may be accomplished by separating the culture medium form the mycelia and thereafter, extracting oosporin from the culture medium. The mycelia is separated from the medium at the desired time or stage by filtration, centrifugation or other conventional methods. The pH of the filtered medium is adjusted to 2.0 to 3.0 and the medium is extracted with a suitable organic solvent.

Extraction of the medium can be performed at any time during fermentation following the appearance of red pigment in the medium. To achieve high yields of the oosporin, it is recommended to extract the pigment after about 7 days of incubation. Extraction of the medium can be performed with ethyl acetate, chloroform or a like organic solvent. The preferred extraction solvent is ethyl acetate.

Extraction of the medium yields an orange solution which is very stable. This orange solution can easily be concentrated by drying to obtain an orange extractant. Vacuum drying evaporation is particularly suitable. The orange pigment extract can be further purified by conventional means, e.g., by dissolving the pigment extract in a suitable solvent (ethyl acetate) and passing through an ion-exchange resin.

The examples which follow are given for illustrative purposes and are not meant to limit the invention described herein. The following abbreviations have been used throughout in describing the invention.

| | |
|---|---|
| CFU | - Colony forming unit |
| CPU | - Conidial spores per prill unit |
| °C | - degree(s) Centigrade |
| FTIR | - Fourier Transfer Image Resolution |
| g | - gram(s) |
| hr | - hour(s) |
| l | - liter(s) |
| μ | - micro |
| % | - percent |
| lb | - pound(s) |
| ft² | - square foot (feet) |
| M | - molar |
| ml | - milliliter |
| N | - normal |
| NMR | - Nuclear Magnetic Resonance |
| PDA | - potato dextrose agar |
| RH | - relative humidity |
| SDA | - Sabouraud's Dextrose Agar |
| SDB | - Sabouraud's Dextrose Broth |
| rpm | - rotation per minute |
| w | - weight |
| vvm | - volume per volume per minute |
| YM | - yeast malt extract media |

Example 1

(Production of Mycelium and Oosporin *Beauveria bassiana* in 20-Liter Batch Fermentor)

The fungus *Beauveria bassiana* (ATCC 74037) was maintained on a slant agar containing 20 g/l malt, 20 g/l glucose, 1 g/l peptone, and 10 g/l agar. The slant was stored at 4°C.

Slants were transferred under sterile conditions to a shake flask medium of 30 g/l glucose, 20 g/l YM, and 20 g/l corn steep liquor. The solution was adjusted to pH 6 before sterilization. After inoculation with the slant fungi, the shake flask was agitated at 30°C for 24 hours on a round shaker at 300 rpm. The shake flask product, mainly blastospores, was used to inoculate a 20-liter fermentor containing 16 liters of production media composed of 60-80 g/l molasses, 20 g/l cotton seed flour, and 20 g/l corn steep liquor. The fermentation pH was controlled to pH 5.3 by adding base (2M NaOH). Aeration was maintained at between 0.8-1.0 vvm, and agitation was maintained at 400-600 rpm. In order to avoid formation of foam, 1.5 ml of Macol® P-2000 antifoam agent (Mazer® chemicals) was added to the fermentation solution. The fermentation was completed after 96-100 hours, and the filamentous mycelium was harvested by centrifugation. The yield of filamentous mycelium obtained was 14 g/l (dry weight).

Samples (100 ml) of the fermentation broth, which were deep red in color, were extracted using ethyl acetate. The broth was filtered to remove mycelium particles and the pH adjusted to 2.0 with acetic acid. The broth was then extracted with ethyl acetate (300 ml). An orange pigment was obtained. Evaporation of the ethyl acetate yielded a dense orange extract which was purified by redissolving the pigment extract with 100 ml of ethyl acetate and loading for purification on a Dowex® ion exchange resin. The isolated red fraction was collected from the column and submitted for analysis by FTIR, NMR and mass spectrophotometer. Analysis concluded that the pigment was oosporin.

Example 2

(Preparation of Biopesticide Formulations)

The mycelium of *Beauveria bassiana* obtained in Example 1 was used to prepare formulations described in Table I. *Beauveria* mycelium was blended briefly and mixed with the described amounts of carriers. The carriers had been previously autoclaved for 1 hour at 121°C with 1 liter of water. Sodium alginate was added and each of the blended mixtures was brought to a total volume of 3 liters and IN NaOH was added to obtain the indicated pH. For prill formation of each mixture, a bath containing 5 liters of a calcium chloride solution at concentration range of 13-27% (pH 6.35-7.00) was used. The blended mycelium/porous carrier mixture was loaded into a prilling column, and the mixture was added dropwise to the coagulation bath to form alginate prill. The prills were submerged in the coagulation bath for 1 hour or longer. The prills were easily removed from the bath by screening through a metallic screen. The wet prills (which contained as much as 80-85% moisture) were loaded onto a fluidized bed dryer and dried at an air temperature below 30°C. The prills were dried to a water content of 6% to 10% w/w.

TABLE I

| Formulations | Carrier | Carrier Amounts amounts (g) | Alginate (g) | Beauveria Mycelium (g) | pH |
|---|---|---|---|---|---|
| 1 | Vermiculite/Bran cotton seed flour | 200/200/50 | 60 | 500 | 6.5 |
| 2 | Vermiculite/Bran cotton seed flour | 100/100/50 | 30 | 250 | 6.5 |

EP 0 570 089 A1

Example 3

(Evaluation in vitro of Prill and Conidial Viability)

The two prill formulations prepared according to Example 2 were evaluated and assessed for viability, condiation and germination during storage.

Prill Viability

Thirty-six prills from each prill formulation were aseptically added to a YM (41 g/l Difco) plate (15 x 100 mm square grid style petri plate, Falcon 1012 Becton Dickinson), the plates were incubated at 25°C for 5 days. The number of prills which grew was recorded and the percentage of viable prills was calculated and recorded in Table II below.

Sporulation assays

Twelve prills were rehydrated with sterile water for 30 minutes, and placed individually aseptically, in the wells of a 96 well plate (Corning Polystyrene #25860). The plate was then incubated at 25°C for 5 days, after the first 24 hours, sterile deionized water was added to each prill using an atomizer. Then the plate was reincubated for 4 more days. At day 5 the spores were harvested from each well by adding 200µl of sterile 0.05% Tween 80 to each well. The contents of each well were carefully pipeted using a sterile inoculating needle (Fisher 13-075-1) into the original test tube containing a final volume of 10 ml Tween 80 solution. The tube was vigorously mixed for 20 seconds and the spores from the resulting suspension were counted using a hemacytometer and recorded in Table II below.

Spore Viability

Based on the spore count as described in Table II below, serial dilutions were prepared resulting in 1 x $10^5$ spores/ml. 100 µl of sterile SDB (30 g/l Difco) was spores/ml. 100 µl of sterile SDB (30 g/l Difco) was added to two columns of a 96 well plate (Corning 25360 Polystyrene). 100 µl of spore suspension was added to each well for a total of eight wells. The plate was incubated at 25°C for 24 hours. Using an inverted phase microscope (Ziess 32x), the number of viable (germ tube present) and the nonviable spores (no germ tube) were counted and recorded in Table II below.

Table II

| Formulation | Storage time (months) | Prill Viability (%) | Conidial Produced (per prill) | Conidial Viability (%) |
|---|---|---|---|---|
| 1 | 0 | 100 | $7.98 \times 10^6$ | 100 |
| | 5 | 100 | $8.44 \times 10^5$ | 100 |
| 2 | 0 | 100 | $2.56 \times 10^6$ | 100 |
| | 6 | 100 | $5.98 \times 10^5$ | 100 |
| | 8 | 100 | $1.25 \times 10^4$ | 100 |

EP 0 570 089 A1

Example 4

(Whitefly Mortality in vitro)

To determine the ability of Beauveria bassiana ATCC 74037 to produce sweet potato whitefly (SPWF) mortality in vitro, ten early SPWF 4th instars (scales) were placed on double tape on a microscope cover slide. Ten cover slides (100 scales) were prepared for the assay and another 10 slides (100 scales) were prepared for the control solution. A fungal solution of $1.66 \times 10^7$ spore/ml was prepared by aseptically harvesting spores from a 14-day old agar plate of Beauveria bassiana in Tween-80® (0.05%) solution. Treatment consisted of dipping the cover slides with scales in the Beauveria bassiana suspension of spore/ml or in the control solution for 10 seconds. After allowing the cover slides to air dry, the slides were placed in a relative humidity chamber where RH = 100% and kept at 25°C under constant light. Tween-80 (0.05%) was used as the control solution. Percent mortality was determined on day 6 to be 92% for the fungus and 8% for the control.

Example 5

(Comparative Whitefly Mortality in vitro)

The activity of Beauveria bassiana ATCC 74037 to produce sweet potato whitefly (SPWF) mortality was compared with that of a commercial preparation of Beauveria bassiana i.e. Naturallis® (Phermone Corp., Arizona). The in vitro test as described in Example 4 was used. The two fungal preparations were prepared. Each of them contained $1 \times 10^6$ spore/ml. All spores were harvested from a potato dextrose agar plate after 14 days of growth with sterile deionized water. The control group of SPWF was treated with deionized water only. Table III indicates the mortality count taken after 7 days.

TABLE III

| Treatment | Mortality % |
|---|---|
| Beauveria bassiana 74037 | 76 |
| Naturallis® | 52 |
| Control | 6 |

Example 6

(Mealy Bugs and Whitefly Mortality in vivo and in vitro)

A fermentation broth containing Beauveria bassiana ATCC 74037 grown in a fermentor as described in Example 1 was used to determine mortality in vivo. Twenty-five mealy bugs were put on leaves of hibiscus plants and another twenty-five sweet potato whiteflies (SPWF) were put on leaves of another hibiscus plant.

The fermentation broth was tested in vitro as described in Example 4. The preparation was brushed on the leaves of the infested plants. Mortality was assessed after day 2 and day 5.

In vitro experiment scales of whitefly showed evidence of infection on day 2 by acquiring a pink color. By day 5 treated scales showed 98% infection with visible fungus growth. Control was 6%. For the in vivo experiment, 49.8% of whitefly scales on treated leaves were infected. These scales were a dark pink or sienna color. Fungi were not visible. This could be due to low humidity conditions in the greenhouse. One of these leaves was taken off the plant and placed in a petri dish with wet filter paper. After 3 days, all infected SPWF scales were showing fungal growth. The scales were then placed on SDA and the red pigment typical of this Beauveria was already visible. All 25 mealy bugs on the leaf were dead seven days after treatment. Several of these were plated on SDA, and the red pigment and fungus appeared after 2 days in all plates.

Example 7

(Sporulation and Germination in Soil)

In order to evaluate the ability of *Beauveria* prills to sporulate and germinate in soil, eight 250 ml Erlenmeyer glass flasks were filled with 15 g of Redi lite soil mix (Terra-Lite®). The flask and the soil were autoclaved for 20 minutes at 121°C. After the soil was cooled to room temperature, 20 vermiculite prills (as prepared and described in Example 2) were added to the soil mixture in the flask. The soil and the prills were shaken well, and 50 ml of sterile deionized water was added to enhance humidity. The soil/prill mixture was incubated at 20°, 25° and 30°C. After 7 and 14 days the number of spores in the soil were evaluated. The spore count was determined by the following method and the results were recorded in Table IV below:

CFU Determination

1. Aseptically mix thoroughly the flask contents using a sterile spoonula.
2. Carefully remove 1 g of wet soil mixture.
3. Make 1:10 serial dilutions using 50 mM phosphate buffer pH 7.0.
4. Plate the sample by adding 100 $\mu$l of sample onto a Rose Bengal plus 100 mg chloramphenicol agar plate and make a spread plate. Prepare triplicate plates.
5. Incubate the plates at 280C.
6. After 5 to 6 days count the fungal colonies present.

TABLE IV

| Prill Formulations | Number of Spores (CFU/g) | | | |
|---|---|---|---|---|
| | Days | 20°C | 25°C | 30°C |
| 1 | 7 | $1.02 \times 10^6$ | $1.41 \times 10^6$ | $9.54 \times 10^5$ |
| | 14 | $1.31 \times 10^6$ | $1.41 \times 10^6$ | $9.07 \times 10^5$ |
| 2 | 7 | $3.67 \times 10^6$ | $5.47 \times 10^6$ | $7.22 \times 10^5$ |

Example 8

(Mortality of Soil Insect Using *Beauveria bassiana* ATCC 74037)

To demonstrate the ability of sporulated *Beauveria bassiana* ATCC 74037 to produce mortality of soil insects, dry prills of *Beauveria bassiana* were introduced to non-sterile (10cc) chandlers soil (dried at room temperature in plastic cups). Three rate of prills were added to the soil in the cup (5 cups per rate) 25 prill per 10 cc soil, 10 prill per 10 cc soil and 5 prill per 10 cc of soil. *Artipus floridanus* (citrus root weevile), 45 day old larvae (third instar) were used for mortality demonstration. Five larvae were introduced to each soil cup. To each cup, which contains the prill and insect, 350 $\mu$l sterile distilled water was added. Two different prill formulations, Formulation 1 and 2 as demonstrated in Example 1, were used and a control which consisted of soil cup without prills and which 350 $\mu$l of sterile water was added. Insect mortality, prill viability and sporulation results are recorded in Table V below.

TABLE V

| Formulation | Prill Viability (%) | Prill Sporulation | Rate[1] | Average Mortality (%) |
|---|---|---|---|---|
| 1 | 100% | $4.59 \times 10^5$ | 25 | 36 |
| | | | 10 | 44.9 |
| | | | 5 | 36 |
| 2 | 100% | $9.74 \times 10^5$ | 25 | 64 |
| | | | 10 | 45.7 |
| | | | 5 | 24 |

[1]No. of prills per 10 cc of soil

## Example 9

(Comparison of Oosporin Production Using *Beauveria bassiana* and *Chaetomium trialaterale)* The *Beauveria bassiana* ATCC 74037 mutant fungus strain of this invention was compared with a known fungal strain which produced oosporin in liquid culture *(Chaetomium trilaterale,* Cole et al., J. Agr. Food Chem., Vol. 22, No. 3, 1974,ATCC 24912). Both strains were inoculated onto petri dishes containing the same agent as described in Example 1. The dishes were incubated at 25°C for 7 days and then visually compared. It is apparent from the visual comparison that the *Beauveria* strain had produced much more dark red pigment that diffused into the agar than the *Chaetomium trilaterale* strain.

## Submerged Fermentaion

The two fungal strains were compared in submerged fermentation. Each strain was transfered into a shake flask containing the medium of Example 1. The flasks were incubated at 28°C and agitated at 150-200 rpm. The flasks were examined usually over 7 days for evidence of color change. After 24 hours the flask containing the *Beauveria* strain had turned red. The color of the flask containing *Chaetomium trilaterale* began to change color only after 3 days.

14

**Claims**

1. A toxin-producing isolate of *Beauveria bassiana* which, when in its essentially biologically pure form, has high virulence against pests and which has the identifying characteristics of *Beauveria bassiana*, culture deposit ATCC 74037 wherein the toxin is oosporin or an analog thereof.

2. A biopesticidal composition for controlling a pest, said composition comprising conidial spores of the fungus *Beauveria bassiana* which has the identifying characteristics of *Beauveria bassiana* culture deposition ATCC 74037 and which produces the toxin oosporin or an analog thereof, in association with an agricultural carrier.

3. The biopesticidal composition of Claim 2 in which the inert carrier is in liquid, powder, granule or particulate form.

4. The biopesticidal composition of Claim 2 in which the carrier is a liquid selected from water or a non-aqueous oil.

5. The biopesticidal composition of Claim 4 in which the non-aqueous oil is selected from cotton seed oil, vegetable oil and mineral oil.

6. The biopesticidal composition of Claim 2 which further comprises a nutrient.

7. A stable, dried granulated biopesticide comprising an inert carrier which is capable of supporting fungal growth and promoting conidial sporulation and a biomass of a fungus *Beauveria bassiana* having the identifying characteristics of *Beauveria bassiana* culture deposit ATCC 74037, which biomass is substantially comprised of mycelium and is prepared by submerged fermentation.

8. The biopesticide of Claim 7 wherein at least about 80% of the biomass is in the form of mycelium.

9. The biopesticide of Claim 7 wherein the mycelium is filamentous in form.

10. The biopesticide of Claim 7 which further comprises a nutrient.

11. The biopesticide of Claim 7 in which said biomass has been sporulated to contain a pesticidally effective amount of active conidial spores of the fungus.

12. The biopesticide of Claim 7 in which said conidial spores are present at a concentration of from about $1 \times 10^7$ to $1 \times 10^8$ conidial spores per biopesticide.

13. A process for preparing a fungal biopesticide for control or prevention of pest infestation comprising
    (a) fermenting one or more species of a toxin-producing fungus *Beauveria bassiana* having the identifying characteristics of *Beauveria bassiana,* culture deposit ATCC 74037, in a culture medium by submerged fermentation to produce a biomass substantially in the form of mycelium;
    (b) harvesting the biomass;
    (c) mixing the biomass with a carrier;
    (d) forming a prill from the biomass/carrier mixture; and
    (e) optionally, drying the prill.

14. The process of Claim 13 which further comprises the following step:
    (f) activating and culturing the prill to produce pathogenic conidial spores; and
    (g) optionally, drying the reactivated prill.

15. The process of Claim 14 which further comprises the following step:
    (h) harvesting the conidial spores from the activated prill.

16. The process of Claim 15 which further comprises the following step:
    (i) pregerminating the harvested conidial spores.

17. The process of Claim 15 which further comprises the following step:
    (j) assaying in vivo the infectivity of the harvested conidial spores.

18. The process of Claim 15 wherein the conidial spores are harvested by washing the activated prill with water or a non-aqueous oil.

19. The process of Claim 18 wherein the non-aqueous oil is selected from cottonseed oil, vegetable oil, peanut oil, palm oil, sesame oil, jojoba oil, coconut oil, soybean oil or mineral oil.

20. A fungal biopesticide prepared by the process of Claim 13.

21. A fungal biopesticide prepared by the process of Claim 14.

22. A fungal biopesticide prepared by the process of Claim 15.

23. A fungal biopesticide prepared by the process of Claim 16.

24. A method for controlling or preventing the pest infestation of a treatment area comprising applying a pesticidally effective amount of the biopesticide of Claims 20, 21, 22 or 23 to the area and activating the biopesticide.

25. The method of Claim 24 in which the pest is a nematode or an insect.

26. The method of Claim 24 in which the pest is selected from whiteflies, aphids, planthoppers, leafhoppers, spittlebugs, scales, thrips, beetles, mealy bugs and spider mites.

27. A process for the fungal production of oosporin in a *Beauveria bassiana* strain having the capacity to produce and accumulate oosporin, said process comprising:
    (a) growing a culture of the fungus *Beauveria bassiana* having the identifying characteristics of *Beauveria bassiana* culture deposit ATCC 74037 in submerged fermentation in a nutrient culture medium comprising a carbon substrate and a nitrogen substrate,
    (b) continuing fermentation to the point that excessive carbon source is present in the culture medium; and
    (c) continuing fermentation thereafter for the purpose of accumulating oosporin and fungal mycelia in the culture medium.

28. The process of Claim 27 in which said nutrient culture medium of step (a) initially comprises about 2.0 to about 5.0 weight percent of a carbon substrate and about 0.5 to about 5.0 weight percent of a nitrogen substrate.

29. The process of Claim 27 in which said submerged fermentation is conducted at between about 20°C and about 28°C.

30. The process of Claim 27 in which the pH of said submerged fermentation is maintained at about 4.5 to 6.0.

31. The process of Claim 27 in which accumulated oosporin is removed form the culture medium by mixing said medium with a non-aqueous solvent for oosporin and separating the resulting non-aqueous red solution.

32. The process of Claim 31 in which said non-aqueous solvent is chloroform, ether or ethyl acetate.

33. The process of Claim 31 in which red pigment is crystallized from said non-aqueous red solution.

34. The process of Claim 31 in which mycelium is removed from the culture medium prior to treatment of the medium with the nonaqueous solvent.

35. A fed-batch fermentation process for the fungal production of oosporin in a *Beauveria bassiana* strain having the capacity to produce and accumulate oosporin, said process comprising:
    (a) growing a culture of the fungus *Beauveria bassiana* having the identifying characteristics of *Beauveria bassiana* culture deposit ATCC 74037 in submerged fermentation in a nutrient culture medium comprising an excess amount of a carbon substrate and a nitrogen substrate,
    (b) continuing fermentation to the point at which red pigment is produced in the culture media,
    (c) adding fresh nutrient culture medium to the fermentation vessel at a rate which maintains an excess amount of carbon substrate,

(d) continuing fed-batch fermentation for the purpose of accumulating oosporin in the culture medium; and

(e) recovering the oosporin from the culture medium.

36. The process of Claim 35 in which said excess amount of carbon substrate is about 4.0 to about 8.0 weight percent of the culture medium.

37. The process of Claim 35 in which said excess amount of nitrogen substrate is about 0.5 to about 5.0 weight percent of the culture medium.

38. The process of Claim 35 in which carbon substrate is maintained at no less than about 1 weight percent in step (c).

39. A process for the production of oosporin comprising cultivating *Beauveria bassiana* having the identifying characteristics of *Beauveria bassiana* culture deposit ATCC 74037 in a culture medium for a sufficient time and at a sufficient temperature to produce oosporin in the medium, and recovering the oosporin.

European Patent
Office

**EUROPEAN SEARCH REPORT**

Application Number

EP 93 25 0133

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | CANADIAN JOURNAL OF MICROBIOLOGY vol. 8, 1962, CA pages 931 - 933 L.C.VINING ET. AL. 'Oosporein production by a strain of Beauveria bassiana originally identified as Amanita muscaria' --- | | A01N63/04 A01N35/06 C12N1/14 ///(C12N1/14, C12R1/645) |
| D,A | CANADIAN JOURNAL OF BOTANY vol. 46, no. 4, April 1968, CA pages 441 - 448 L.C.VINING ET. AL. 'Pigments of the genus Beauveria' --- | | |
| A | EP-A-0 387 640 (W.R.GRACE & CO.-CONN.) --- | | |
| A | FR-A-2 480 304 (SPOFA SPOJENE PODNIKY PRO ZDRAVOTNICKOU VYROBU) --- | | |
| A | CHEMICAL PATENTS INDEX, DOCUMENTATION ABSTRACTS JOURNAL Section Ch, Week 9007, 11 April 1990 Derwent Publications Ltd., London, GB; Class C, AN 049216 & JP-A-02 003 604 (NIPPON FERTILISER KK) 9 January 1991 --- | | TECHNICAL FIELDS SEARCHED (Int. Cl.5) A01N |
| A | CENTRAL PATENTS INDEX, BASIC ABSTRACTS JOURNAL Section Ch, Week 8204, 24 March 1982 Derwent Publications Ltd., London, GB; Class C, AN 07202/E04 & SU-A-572 163 (BIOL RES INST) 7 September 1981 ----- | | |

The present search report has been drawn up for all claims

| Place of search | Date of completion of the search | Examiner |
|---|---|---|
| THE HAGUE | 26 AUGUST 1993 | DONOVAN T.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0401)